# EUROPEAN PATENT APPLICATION

(11) **EP 2 477 030 A2**
(43) Date of publication of application: **18.07.2012**
(21) Application number: 10763733.2
(22) Date of filing: 07.09.2010
(51) Int. Cl.: G01N 33/68

(54) **NOVEL BIOMARKERS OF DISEASE HISTOPATHOLOGY**

(30) Priority: 07.09.2009 ES 200930661; 25.01.2010 ES 201030090
(71) Applicant: Fundación del Hospital Nacional de Parapléjicos Para la Investigación y la Integración, 45071 Toledo (ES)
(72) Inventor: DE CASTRO SOUBRIET, Fernando, 45071 Toledo (ES); CLEMENTE LÓPEZ, Diego, 45071 Toledo (ES); ORTEGA MUÑOZ, María Cristina, 45071 Toledo (ES); ARENZANA SANAGÉRICO, Francisco Javier, 45071 Toledo (ES)
(74) Representative: Pons Ariño, Angel
(86) International application number: PCT/ES2010/070584
(87) International publication number: WO 2011/045456

(57) **Abstract**

Use of the FGF-2 and anosmin-1 proteins for predicting the histopathology of the lesions of a patient suffering from a central nervous system (CNS) demyelinating disease by means of the detection of the amount of said proteins in a sample of isolated biological fluid. Furthermore, the present invention relates to a method for ascertaining the histopathological characteristics of the lesions of a patient suffering from a CNS demyelinating disease and to a kit for implementing said method. Preferably, the CNS demyelinating disease is multiple sclerosis and the biological fluid is cerebrospinal fluid (CSF)..

## Description

The present invention relates to the field of Biomedicine. Specifically, it refers to the use of proteins FGF-2 and Anosmin-1, to predict the histopathology of the lesions of an individual with a demyelinating disease of the central nervous system (CNS) by detecting the amount of such proteins in an isolated biological fluid sample. Furthermore, this invention relates to a method to understand the histopathological features of the lesions of an individual with a demyelinating disease of the CNS by means of a kit for the implementation of this method. Preferably, the CNS demyelinating disease is multiple sclerosis and the biological fluid is cerebrospinal fluid (CSF).

### STATE OF THE ART

Demyelinating diseases are a serious clinical and social problem. They are a set of neurological diseases in which, for various reasons, there is damage to the myelin sheath coating neuronal axons, which protects and facilitates nerve impulse conduction. The most prevalent of these diseases is multiple sclerosis.

In many cases, multiple sclerosis begins as a disease with outbreaks (or acute phases) with the typical neurological alterations of this pathology. Each outbreak is followed by a phase of remission after which the patient may or may not suffer after-effects with different severity degree. In most cases, after a phase of multiple sclerosis outbreaks, the disease derives to a secondary progressive form in which the patient suffers continuous neurological deficits, and disability increases steadily and progressively (cf. Confavreux et al., N. Eng. J. Med. 2000, vol. 343, pp. 1430-1438).

In the case of multiple sclerosis, oligodendrocytes, myelin-producing cells in the CNS, die in the injured areas, also known as demyelinating plaques. Oligodendrocytes originate during pre- and postnatal development from oligodendrocyte precursor cells, cells that also exist in the adult brain of all mammals. In response to injury, oligodendrocyte precursor cells are mobilized to the injured brain area, although, depending on the stage of evolution of the lesion, they may or may not enter and repair the myelin sheaths (cf. Wolswijk, J . Neurosci. 1998, vol. 18, pp. 601-609, Chang et al., J. Neurosci. 2000, vol. 20, pp. 6404-6412, Chang et al., N. Engl. J. Med. 2002 , vol. 346, pp. 165-173; Reynolds et al., J. Neurocytol. 2002, vol. 31, pp. 523-536; Chandran et al., Philos. Trans. R. Soc. Lond. B. Biol . Sci 2008, vol. 363, pp. 171-183).

In recent years, existing therapies to ease (not cure) multiple sclerosis, try to reduce the existing level of inflammation in the CNS, but are not aimed at the mobilization of oligodendrocyte precursor cells into the area needing repair. Therefore, cell therapy, either by using stem cells, mesenchymal cells or oligodendrocyte precursor cells themselves (including endogenous ones), is a hopeful future for the treatment of multiple sclerosis. Therefore, it is very important to know in each multiple sclerosis patient the balance between the lesions in which remyelination is possible and those where it is not when choosing the type of treatment (reliever of inflammatory effects versus restorative cell therapy).

Demyelinating lesions observed in the CNS of a patient with multiple sclerosis can be classified histopathologically into three types according to the distribution of myelin-destroying cells (lymphocytes, macrophages and microglia) and the degree of myelin destruction (cf. Trapp et al., J. Neuroimmunol., 1999, vol. 98, pp. 49-56, Benito et al., J. Neurosci., 2007, vol. 27, pp. 2396-2402):
1.- ACTIVE LESIONS: lesions in which demyelination can be complete or incomplete and whose interior is filled with myelin-destroying cells, evenly distributed throughout the area of injury. In these lesions, remyelination is possible (in fact, the so-called "shadow plaques" form where there is a partial remyelination), with oligodendrocyte precursor cells inside (they can therefore enter) and they are considered from a chronological point of view as the first to appear.
2.- CHRONIC LESIONS: lesions in which demyelination is complete, the interior is devoid of myelin-destroying cells, which accumulate in the peripheral area of injury. Remyelination in these lesions (repair of the myelin sheath) is very unlikely and is restricted to the edge of the lesion, there are no oligodendrocyte precursor cells inside (they are unable to enter or, if they do, they die); and they are considered the next step after active lesions.
3.- INACTIVE LESIONS: lesions in which demyelination is complete and myelin-destroying cells are no longer present either inside or on the periphery of the lesion. Remyelination in these lesions is not feasible, also lacking oligodendrocyte precursor cells inside, and they are considered the final step in a demyelinating lesion.

Because the inflammatory environment of multiple sclerosis is continuous throughout the disease, when performing a cellular therapy, the chances of success will be greater in those patients whose injuries are exclusively of an ACTIVE type (and thus prone to be repaired.) However, the only reliable evidence of the type of injury present in the CNS of a multiple sclerosis patient is a histopathological analysis. For obvious reasons, it is the most invasive method and in almost all cases it is only feasible (and therefore useful) post-mortem. Images obtained by nuclear magnetic resonance, although to date the existing less invasive and more sensitive method, do not specify the type of injuries of a multiple sclerosis patient, their developmental state or their possible evolution, not only in the white matter (where the vast majority of the myelin sheaths are found) but also in the grey matter, which could trigger damage to the neurons themselves (Brex et al., N. Eng. J. Med. 2002 , vol. 346, pp. 158-164, Sepulchre et al., Arch Neurol., 2009, vol. 66, pp. 173-179; Weiner, Ann. Neurol., 2009, vol. 65, pp. 239 - 248). Although the aforementioned work of Sepulchre et al. (Sepulchre et al., Arch Neurol., 2009, vol. 66, pp. 173-179) is an important step in establishing a causal relationship between the presence of white matter lesions and atrophy of the grey matter in multiple sclerosis patients, in the editorial by Reichert et al. (Reichert et al., Arch Neurol., 2009, vol. 66, pp. 159-160) and in the same volume of Archives of Neurology in which these relationships are analysed, the conclusion is clearly stated that "until more sensitive measures of brain injury are not achieved by nuclear magnetic resonance, the interesting work by Sepulchre et al. must be corroborated by post-mortem analysis of brain tissue of multiple sclerosis patients". It is therefore necessary, by using non-invasive techniques, to find bio-markers (present in CSF or serum of multiple sclerosis patients) that can be used to determine the type of lesions in the CNS, the more efficient treatment for the repair of these lesions (including the design of clinical trials) or the possible evolution of the disease, both in the white matter and grey matter.

Sarchielli et al. describe the use of the FGF-2 measurement in the diagnosis/prognosis of multiple sclerosis, though they only associate the level of FGF-2 to the clinical variants of the disease, but do not study or mention the histopathology of the lesions (Neuroscience Letters 435 (2008) 223-228).

Knowing the histopathology of the demyelinating lesions of an individual is extremely useful. By the uses and methods described in the present invention, these lesions can be detected before the individual had symptoms and was diagnosed.

### DESCRIPTION OF THE INVENTION

The authors of this invention provide a new tool to understand, through the FGF-2 and/or Anosmin-1 levels in patients' cerebrospinal fluid (CSF), the degree of their brain injuries, the environment enabling or inhibiting the migration of oligodendrocyte precursor cells, restorative cells of the myelin sheath, the prognosis of the possible evolution of the disease towards neuronal damage in the grey matter and the identification of the appropriate treatment for the same (including the design of clinical trials).

The present invention therefore provides a non-invasive tool for the histopathological cataloguing of lesions in the white matter of multiple sclerosis patients by only analysing the FGF-2 and/or Anosmin-1 level in CSF.

As demonstrated in the examples of the present invention, the molecule FGF-2 and/or Anosmin-1 levels in the CSF reflect the type or types of lesions of the central nervous system (CNS) of a multiple sclerosis patient without need for more invasive tests or diagnostic errors, with the same level of certainty as if a biopsy was performed, the only reliable test of the degree of damage found in the CNS of any neurological patient, including patients with multiple sclerosis. Multiple sclerosis patients who have FGF-2 and/or Anosmin-1 levels similar to those found in control individuals, present only numerous small lesions of the ACTIVE type. However, those patients with a significantly higher FGF-2 and/or Anosmin-1 level in CSF than the level in control subjects (at least double -twice as much-), present large CHRONIC or INACTIVE-type lesions in the white matter, fewer in number than in the previous cases, but greater in size.

Furthermore, the present invention shows that the FGF-2 and/or Anosmin-1 levels in CSF reflect the status of the molecular environment, enabling or inhibiting the arrival of the restorative cells of the myelin sheath in demyelinating lesions within the white matter.

In those patients with FGF-2 and/or Anosmin-1 levels in the CSF similar to those found in control patients, ACTIVE lesions are associated with the presence of the motogenic and chemoattractant molecule of the oligodendrocyte precursor cells, FGF-2, expressed only by certain microglia/macrophage cells within the lesion, but FGF-2 or its receptor FGFR1 are never detected in the vicinity of the demyelination plaque. The presence of molecules which, such as Anosmin-1, prevent or hinder the migration of oligodendrocyte precursor cells, is not detected within active lesions, clearly indicating that these lesions present the right circumstances for a possible remyelination and, therefore, for their repair. In the case of multiple sclerosis patients with a FGF-2 level in CSF at least twice as high as this level in non demyelination conditions, their CHRONIC and/or INACTIVE injuries histopathologically present both abundant macrophages/microglia expressing FGF -2, and oligodendrocyte precursors expressing its receptor FGFR1 around them; the FGF-2 acts as a motogenic and chemoattractant molecule in an attempt to attract oligodendrocyte precursors towards the demyelinated area to be repaired. However, CHRONIC and/or INACTIVE injuries found in multiple sclerosis patients with these FGF-2 levels in CSF, show a high presence of Anosmin-1 in their interior, which is detrimental (inhibitory, chemorepellant) for migration of oligodendrocyte precursors, restorative cells of the injury, so they fail to enter and therefore the myelin sheath repair is not carried out.

Moreover, measurements of the FGF-2 and/or Anosmin-1 levels in the CSF of MULTIPLE SCLEROSIS patients reflect changes in the grey matter, both at the cellular level and the state of blood-brain barrier of grey matter, thus prognosticating the onset of neuronal damage.

In multiple sclerosis patients with a FGF-2 and/or Anosmin-1 level in CSF similar to individuals without demyelinating lesions, their grey matter shows no FGF-2 or Anosmin-1 nor apparent alterations of the blood-brain barrier, which means less risk for the neuronal bodies. By contrast, patients with FGF-2 and/or Anosmin-1 levels in CSF significantly higher (at least twice as high) than in non demyelination cases, show perivascular astrocytes which produce FGF-2 and Anosmin-1, and also express their common receptor FGFR1, in places where the blood-brain barrier is compromised, in an attempt to rebuild it. This damage to the blood-brain barrier results, at further stages of the disease, in lesions in the neuronal bodies in these areas. This can cause more severe damage of the motor functions or even alterations in the cognitive functions of the subject.

Therefore, the measurement of the FGF-2 and/or Anosmin-1 levels in CSF serves as a biomarker of histopathological damage level, of the susceptibility to repair by using oligodendrocyte precursors, as well as the damage of the grey matter. A biomarker is a substance that can be measured and whose values can be objectively associated to a biological state, such as suffering from a disease or not. The biomarkers are also used in the study of the response to treatment.

Therefore, a first aspect of the invention relates to the use of proteins FGF-2, Anosmin-1 or both, to predict the histopathology of the lesions of an individual with a possible demyelinating disease, preferably of the CNS, by detecting the amount of such proteins in a sample of isolated biological fluid.

In another preferred embodiment of the invention, FGF-2 and Anosmin-1 proteins were used to predict the histopathology of the lesions of an individual with a possible demyelinating disease, preferably of the CNS, by detecting the amount of such proteins in an isolated biological fluid sample.

In a preferred embodiment, the FGF-2 and Anosmin-1 proteins were used as remyelination biomarkers in a hypothetical cell therapy in a patient with a demyelinating disease. These proteins may therefore serve as biomarkers to monitor the development of a patient diagnosed with a demyelinating disease and to monitor progress during and after treatment, as the levels of these proteins are indicative of the patient's state of myelination. A patient can be treated, for example, but not limited to, with a cell therapy aimed at myelin regeneration.

In a preferred embodiment of the invention, the FGF-2 or Anosmin-1 proteins, or both, are used to predict the presence of at least one CHRONIC or INACTIVE demyelinating lesion. In a preferred embodiment, the FGF-2 protein is used. In a preferred embodiment, the Anosmin-1 protein is used. In a preferred embodiment, a combination of both is used.

In a preferred embodiment of the invention, the Anosmin-1 protein is used as a biomarker for the detection of demyelinating disease, preferably of the CNS. In a preferred embodiment of the invention, the Anosmin-1 protein is used to detect a demyelinating disease in an isolated biological sample, preferably in an isolated biological fluid.

As used in this description, the terms "isolated biological fluid sample" or simply "biological sample" refer to an individual's biological fluid, obtained by any method known by one skilled in the art that serves for that purpose.

The biological fluid may include fluids excreted or secreted from the body, as well as fluids that normally are not. Biological fluid may include amniotic fluid surrounding the foetus, aqueous humor, interstitial fluid, lymph, breast milk, mucus (including nasal drainage and phlegm), saliva, sebum (skin oil), sweat, urine, semen and pericardial fluid. The isolated biological sample can be, for example, but not limited to, fresh, frozen or fixed.

In a preferred embodiment, the isolated biological fluid sample is any bodily fluid, preferably selected from the list that includes: CSF, blood, blood serum, blood plasma and tears. In a more preferred embodiment, the isolated sample of biological fluid is CSF.

The term "subject" as used in the description applies to animals, preferably mammals and most preferably, humans. Preferably, this term refers to an individual who has been diagnosed with a CNS demyelinating disease. A subject may be, therefore, a patient suffering from a demyelinating disease or a healthy individual.

As used in this description, the term "CNS demyelinating disease" refers to a disease characterised by loss or dysfunction of myelin in the CNS.

In a preferred embodiment, the demyelinating disease affects the CNS and is selected from the list that includes: multiple sclerosis, Devic's neuromyelitis optica, acute disseminated encephalitis, acute transverse myelitis, acute or sub-acute hemorrhagic leukoencephalitis, acute disseminated, diffuse sclerosis, central demyelination of the corpus callosum, central pontine myelinolysis, sub-acute necrotising myelitis, concentric sclerosis, adrenoleukodystrophy, Alexander disease, Canavan disease, Krabbe disease and Zellweger syndrome.

Multiple sclerosis is the most common CNS demyelinating disease. Pathologic findings include multiple well-defined areas of demyelination throughout the CNS white matter. Clinical manifestations include visual loss, abnormal extraocular movements, paresthesia, numbness, weakness, dysarthria, spasticity, ataxia, and bladder dysfunction. In a more preferred embodiment, the CNS demyelinating disease is multiple sclerosis, in any of its clinical variants.

The most common clinical variants of the disease are: relapsing-remitting multiple sclerosis, Secondary Progressive multiple sclerosis, Primary Progressive Multiple Sclerosis, Progressive Relapsing multiple sclerosis and clinically isolated syndrome (CIS).

Relapsing-remitting Multiple Sclerosis is the most common clinical variant of Multiple Sclerosis characterised by outbreaks or acute and recurrent exacerbations of neurological dysfunction followed by partial or complete recoveries. Common clinical manifestations include loss of visual, motor, sensory or bladder functions. Acute demyelination episodes can occur anywhere in the CNS, commonly affecting the optic nerves, spinal cord, brain, brain stem and cerebellum.

Secondary Progressive Multiple Sclerosis is the usual clinical course of relapsing-remitting Multiple Sclerosis; there is a decline in the number of outbreaks and disability progression.

Primary Progressive Multiple Sclerosis is characterised by a gradual disability progression from the beginning; patients do not experience relapses, but a steady functional decline from the onset of disease.

Finally, Progressive Relapsing Multiple Sclerosis, much less common than the previous ones, begins with a primary progressive course and overlapping outbreaks.

In an even more preferred embodiment, the CNS demyelinating disease is the primary progressive or secondary progressive clinical variant of multiple sclerosis.

In another preferred embodiment, the demyelinating disease affects the peripheral nervous system (PNS) and is, preferably, the Guillain-Barré syndrome.

In a preferred embodiment, both proteins, FGF-2 and Anosmin-1, are used as biomarkers for the detection of demyelinating disease, preferably of the CNS. Preferably, the levels of both proteins are detected in an isolated biological sample, preferably an isolated biological fluid.

A second aspect of the invention relates to a method for obtaining useful data for the detection of demyelinating disease or to predict the histopathology of the lesions of a subject or a patient with a CNS demyelinating disease (henceforth, method of the invention) comprising the following steps:
a) obtaining an isolated biological sample from a subject, preferably isolated biological fluid.
b) detecting the amount of FGF-2 and/or Anosmin-1 protein in the biological sample described in step (a); and
c) comparing the amount detected in step (b) with a reference quantity.

In a preferred embodiment, the method of the invention comprises the following step:
d) predicting the presence of at least one chronic or inactive lesion in the white matter and/or the existence of blood-brain barrier changes in the grey matter in the individual.

Steps (b) and/or (c) of the method of invention can be wholly or partially automated, for example, through robotic sensor equipment for the detection of the amount in step (b) or the computerised comparison in step (c). In addition to the above steps additional steps may be included, for example related to the pre-treatment of the sample or the assessment of the results obtained by this method.

The term "predict", as used in the present invention relates to the ability to classify the lesions of a subject with a demyelinating disease of the CNS according to their histopathologic characteristics, for example, the type of lesions present in the white matter or the existence of blood-brain barrier alterations in grey matter, when using a sample-classification method based on the analysis of the amount of FGF-2 and/or Anosmin-1 protein and on the comparison of the amount detected with respect to a reference quantity. This discrimination as understood by an expert in the field is not intended to be correct in 100% of the samples analysed. However, it requires that a statistically significant number of the samples are classified correctly. The amount that is statistically significant can be established by one skilled in the art by using various statistical tools, for example, but not limited to, by determining confidence intervals, *p* value determination, Student's test or Fisher's discriminant functions. Preferably, the confidence intervals are at least 90%, at least 95%, at least 97%, at least 98% or at least 99%. Preferably, the *p* value is less than 0.1, 0.05, 0.01, 0.005 or 0.0001. Preferably, the present invention can correctly detect the disease in at least 60%, in at least 70%, in at least 80%, or at least 90% of the subjects of a particular group or population analysed.

The term "white matter", as used in this description refers to part of the CNS and, more preferably, the brain, composed mainly of myelinated nerve fibres. The term "grey matter", as used in this description refers to part of the CNS and, more preferably, the brain, composed primarily of dendrites and neuronal bodies, which have no myelin.

The fibroblast growth factor-2 protein (FGF-2), also called basic fibroblast growth factor (bFGF), is encoded by a FGF-2 gene belonging to the family of fibroblast growth factors. It has been involved in various biological processes such as CNS development, wound repair and tumour growth.

The term "FGF-2 protein" as used in this description, refers to any protein resulting from the expression of the human FGF-2 gene (GenID: 2247). The prototypical amino acid sequence of the FGF-2 protein is SEQ ID NO: 1 (NP_001997.5). Therefore, the term "FGF-2 protein" also refers to the protein whose amino acid sequence is SEQ ID NO: 1, a variant thereof or a fragment of the above, provided that said variant or fragment is functionally equivalent.

The Anosmin-1 extracellular matrix protein, also known as "Kallmann syndrome protein" and "Adhesion molecule-like X-linked" is encoded in the human gene KAL1 and has been implicated in diverse biological processes both in CNS development and in pathological conditions (Kallmann syndrome, cancer).

The term "Anosmin-1 protein", as used in this description, refers to any protein resulting from human gene KAL1 expression (GenID: 3730). The prototypical amino acid sequence of the Anosmin-1 protein is SEQ ID NO: 2 (NP_000207.2). Therefore, the term "Anosmin-1 protein" also refers to the protein whose amino acid sequence is SEQ ID NO: 2, a variant thereof or a fragment of the above, provided that said variant or fragment is functionally equivalent.

In the sense used in this description, the term "variant" refers to a protein substantially homologous to FGF-2 and/or Anosmin-1 proteins. In general, a variant includes additions, deletions or substitutions of amino acids. The term "variant" also includes protein resulting from post-translational modifications such as, without limitation, glycosylation, phosphorylation or methylation.

As used herein, a protein is "substantially homologous to the FGF-2 or Anosmin-1 protein" when its amino acid sequence is in good alignment with the amino acid sequences SEQ ID NO: 1 and/or SEQ ID NO: 2 , i.e. when its amino acid sequence shows a degree of identity with respect to the amino acid sequence SEQ ID NO: 1, of at least 50%, typically of at least 80%, advantageously of at least 85%, preferably of at least 90%, more preferably of at least 95%, and even more preferably of at least 99%.

The term "identity" as used in this description refers to the proportion of identical amino acids between two amino acid sequences being compared. The percentage of identity between two sequences can be easily identified by one skilled in the art, for example, with the help of a suitable computer program for comparing sequences.

The term "fragment" as used in this description refers to a portion of the FGF-2 and Anosmin-1 proteins or one of their variants

The term "functionally equivalent", as used herein, means that the variant or fragment in question essentially have the immunological properties described in this document. These immunological properties can be determined by conventional methods such as those described in the examples which accompany this description.

The phrase "detecting the amount of FGF-2 and/or Anosmin-1 protein" in an isolated biological fluid sample from an individual, as used in the description, refers to the amount or concentration measure, preferably semi-quantitative or quantitative. Measurement can be performed directly or indirectly. Direct measurement refers to the amount or concentration measure of the FGF-2 and/or Anosmin-1 proteins, based on a signal obtained directly from the protein, and which is directly correlated with the number of protein molecules present in the sample. This signal - which can also be referred to as intensity signal - can be obtained, for example, by measuring an intensity value of a chemical or physical property of the FGF-2 and/or Anosmin-1 proteins. Indirect measurements include the measurement obtained from a secondary component (e.g., a different component from the gene expression product) and a biological measurement system (e.g. the measurement of cellular responses, ligands, "tags" or enzymatic reaction products).

The term "amount", as used in the description refers but is not limited to the absolute or relative amount of proteins, and any other value or parameter associated with the same or which may result from these. Such values or parameters comprise intensity values of the signal obtained from either physical or chemical properties of the protein, obtained by direct measurement, for example, intensity values in an immunoassay, mass spectroscopy or a nuclear magnetic resonance. Additionally, these values or parameters include those obtained by indirect measurement, for example, any of the measurement systems described elsewhere in this document.

The term "comparison" as used in the description refers but is not limited to the comparison of the amount of FGF-2 and/or Anosmin-1 proteins in the biological sample obtained in step (a) of the method of invention, with an amount of reference of FGF-2 and/or Anosmin-1 proteins. The comparison described in paragraph (b) of the method of the present invention can be computer assisted or performed manually.

The term "reference amount" as used in the description refers to the amount of expression product needed to classify the injury of a subject with a demyelinating disease of the CNS, according to the presence or absence of at least one chronic or inactive injury in the white matter and/or whether or not the blood-brain barrier changes in the grey matter.

The reference amount can be calculated by statistical methods known in the state of the art from a population of subjects for which the amount of expression of FGF-2 and/or Anosmin-1 present in the CSF is known, as well as the histopathological characteristics of lesions in the white matter or grey matter. The detection of the amount of FGF-2 and/or Anosmin-1 expression present in the CSF and the analysis of the histopathological features of lesions in the white matter or grey matter of the population of subjects can be done by any procedure described in the prior art, including but not limited to, those described in the examples of this description.

Preferably, the reference quantity defines a threshold amount. The appropriate reference or threshold amounts can be determined by the method of the present invention using a reference sample that can be analysed, for example, simultaneously or consecutively, along with the problem biological sample. More preferably, the threshold amount can be derived from the normal distribution limits of a physiological amount found in a population of control subjects.

In a preferred embodiment, in step (d) of the method of the invention, a greater amount of FGF-2 and/or Anosmin-1 protein detected in step (b) than the reference amount to which it is compared in step (c) is indicative of at least one CHRONIC or INACTIVE injury in the white matter and/or the existence of blood-brain barrier changes in the grey matter. In a preferred embodiment, the amount of FGF-2 and the amount of Anosmin-1 in control subjects is, on average, at least twice as low as the average amount detected in patients with at least one chronic or inactive demyelinating lesion.

In a preferred embodiment of the method of the invention, the isolated biological sample in step (a) is a biological body fluid, and preferably it is selected from the list that includes: CSF, blood, blood serum, blood plasma and tears. More preferably, the sample is CSF.

The method of invention is preferably used to detect or predict the histopathology associated with the demyelinating diseases described above.

In a preferred embodiment, the detection of the amount of FGF-2 and/or Anosmin-1 proteins was performed by incubation with a specific antibody in an immunoassay. The term "immunoassay" as used in this description, refers to any analytical technique that is based on the reaction of the combination of an antibody with the sample. Examples of immunoassays known in the prior art are, for example, but not limited to: immunoblot, enzyme-linked immunosorbent assay (ELISA) or protein microarrays.

The term "antibody" as used in this report, refers to immunoglobulin molecules and immunologically active portions of immunoglobulin molecules, i.e. molecules that contain an antigen binding site that specifically binds (immunoreaction) with the FGF-2 or Anosmin-1 proteins. Examples of portions of immunologically active immunoglobulin molecules include F (ab) and F (ab ') 2 that can be generated by treating the antibody with an enzyme such as pepsin. The antibodies may be polyclonal (typically including different antibodies directed against different determinants or epitopes) or monoclonal (directed against a single determinant in the antigen). The monoclonal antibody can be altered biochemically, by genetic manipulation, or it may be synthetic, the antibody perhaps lacking, in whole or in parts, parts that are not necessary for the recognition of the FGF-2 and/or Anosmin -1 proteins and being replaced by others that communicate additional advantageous properties to the antibody. The antibody may also be recombinant, chimeric, humanised, synthetic or a combination of any of the foregoing. A "recombinant antibody or polypeptide (rAC) is an antibody that has been produced in a host cell that has been transformed or transfected with nucleic acid encoding the polypeptide, or the polypeptide is produced as a result of homologous recombination. Antibodies that recognise the FGF-2 or Anosmin-1 protein are known in the prior art, such as those described, but not limited to, in the examples of this description.

In a preferred embodiment, the immunoassay is an immunoblot or Western blot. To perform an immunoblot, a protein extract is obtained from an isolated biological fluid sample from a subject and proteins are separated in a supporting medium capable of retaining them by electrophoresis. After separation, proteins are transferred to a different medium where they can be detected using specific antibodies that recognise the FGF-2 or Anosmin-1 proteins.

Electrophoresis is a kinetic-based analytical separation technique based on the movement or migration of macromolecules dissolved in a certain medium (electrophoresis buffer), through a matrix or grid support as a result of the action of an electric field. The behaviour of the molecule is given by its electrophoretic mobility and the latter by the charge, size and shape of it. The higher the charge/size the faster an ion migrates within the electric field. There are many variations of this technique in terms of equipment used, support and physical-chemical conditions in which the separation is carried out. Some variations of this technique are, for example, but not limited to, capillary electrophoresis, paper electrophoresis, agarose gel electrophoresis, polyacrylamide gel electrophoresis (PAGE), isoelectric focusing or two-dimensional electrophoresis (2D-PAGE). Electrophoresis can be carried out in non-denaturing or denaturing conditions.

Once the proteins have been separated by electrophoresis, and prior to detection, the proteins are transferred to a support or a membrane, for example, but not limited to, PDVF, nitrocellulose or cellulose acetate. This membrane is hybridised with a specific antibody (also called primary antibody) which recognises the FGF-2 or Anosmin-1 proteins. Then the membrane is hybridised with an antibody (also called secondary antibody) capable of specifically recognising the primary antibody and which is conjugated or linked with a marker compound. In an alternative embodiment, it is the antibody that recognises the FGF-2 or Anosmin-1 proteins the one which is linked or joined to a marker compound, and the use a secondary antibody is not necessary. Once the protein has been detected, its relative molecular size can be determined, comparing its migration with the migration of a control protein detected simultaneously, preferably in the same medium, which has a known size.

In another preferred embodiment, the immunoassay is an enzyme-linked immunosorbent assay or ELISA. The ELISA is based on the premise that an immunoreagent (biological sample antigen or antibody) can be immobilised in a solid medium, then putting the system in contact with a fluid phase containing the additional reagent which can bind to a marker compound.

There are different types of ELISA. In the direct ELISA or simple two-layer ELISA, the solid medium is coated with the biological sample and incubated with an antibody that recognises the FGF-2 or Anosmin-1 proteins, conjugated or attached to a marker compound. In the indirect ELISA, the solid medium is coated with the biological sample and incubated with a primary antibody that recognises the FGF-2 or Anosmin-1 proteins and then a secondary antibody that recognises the primary antibody, conjugated or attached to a marker compound. In the sandwich ELISA or antigen capture assay and detection by immune complexes, the well is coated with a first antibody that recognises and binds to the FGF-2 protein or Anosmin-1, the problem biological sample is applied, so the FGF-2 protein or Anosmin-1 will be retained in the well having been recognised by the first antibody, and then a second antibody is applied that recognises the FGF-2 or Anosmin-1 proteins, bound to a marker compound.

In another preferred embodiment, the immunoassay is a protein microarray whereby a collection of proteins are immobilised in a solid medium in a regular and preestablished pattern. There are several important factors to consider in the design of protein microarrays, such as, for example, the nature of the medium in which the immobilisation takes place, the technique of protein immobilisation, the microarray format, the capture agent or the detection method used. Different formats, media and techniques that can be used to achieve this preferred aspect of first and second methods of the invention are known in the prior art.

The term "marker compound", as used in this description refers to a compound capable of giving rise to a chromogenic, fluorogenic, radioactive and/or chemiluminescent signal allowing the detection and quantification of the amount of FGF-2 and/or Anosmin-1 proteins. The marker compound is selected from the list comprising radioisotopes, enzymes, fluorophores or any molecule capable of being combined with another molecule or detected and/or quantified directly. This marker compound can bind to the antibody directly, or through another compound. Some examples of marker compounds that bind directly are, without limitation, enzymes such as alkaline phosphatase or peroxidase, radioactive isotopes such as 33P or 35S, fluorophores such as fluorescein or metal particles, for direct detection by colorimetry, auto-radiography, fluorimetry or metallography respectively.

In a preferred embodiment of the method of the invention, the FGF-2 and Anosmin-1 protein levels are detected in isolated biological samples from the same patient or subject, and these levels are compared with the respective reference levels. The biological sample in which the FGF-2 levels are detected may be the same biological sample in which Anosmin-1 levels are detected or a different one.

The present invention also relates to a kit that contains the information required to carry out the method of invention.

Therefore, a third aspect of the present invention relates to a kit for carrying out any of the embodiments of the method of the invention.

The kit can contain all those reagents needed to detect the amount of FGF-2 and/or Anosmin-1 proteins by means of any of the methods described earlier in this document, for example, but not limited to, specific antibodies against FGF-2 and/or Anosmin-1 proteins, secondary antibodies or positive and/or negative controls. The kit may also include, without limitation, buffers, protein extraction solutions, contamination prevention agents, protein degradation inhibitors, etc. In addition, the kit may include all media and containers required for its implementation and optimisation. Preferably, the kit also includes instructions for carrying out the method of the invention.

The methods and kits described in the present invention enable the classification of the lesions of a subject with a demyelinating disease of the CNS based on the presence or absence of at least one chronic or inactive lesion in the white matter. The methods and kits described in this invention are therefore useful to select the type of treatment for a patient with this disease (including clinical trials), because high levels of this molecule in the CSF indicate a greater degree of damage and, therefore, the impossibility of remyelination, including through the use of restorative cell therapy based on the use of transplantation with oligodendrocyte precursors (or other cell types), whichever the source (autotransplantation, donor, etc..). However, FGF-2 and/or Anosmin-1 levels similar to those found in non demyelinating conditions indicate that lesions in these patients' CNS are capable of regeneration, and therefore it is possible to use, with a greater expectation of success, cellular therapies to help such repair by using transplantation of oligodendrocyte precursors (and other types of cells) of the same individual or a donor, or pharmacological therapies for the potentiation of endogenous oligodendrocyte precursors.

The methods and kits described in the present invention enable the classification of the lesions of a subject with a CNS demyelinating disease depending on whether or not there are blood-brain barrier alterations in the grey matter. The methods and kits described in this invention are therefore useful for predicting the evolution of a patient with a demyelinating disease towards a possible damage to neurons, something that would happen in the case of finding a significantly higher FGF-2 and/or Anosmin-1 level in the CSF than that found in non demyelinating conditions. Therefore, this measure in the patient's CSF is the basis for the use of other therapies (including new clinical trial designs) that lead to neuroprotection, not only neurorepair, i.e. focused on protecting neurons rather than repairing the myelin sheaths surrounding the axons.

A preferred embodiment of the aspects of the present invention relates to the use of the FGF-2 protein.

A preferred embodiment of the aspects of the present invention relates to the use of the Anosmin-1 protein.

A preferred embodiment of the aspects of the present invention relates to the use of the combination of the FGF-2 protein and the Anosmin-1 protein.

Throughout the description and claims the word "comprise" and its variations are not intended to exclude other technical features, additives, components or steps. For those skilled in the art, other objects, advantages and features of the invention will be inferred partly from the description and partly from the practice of the invention. The following figures and examples are provided by way of illustration, and are not intended to limit the present invention.

### DESCRIPTION OF THE FIGURES

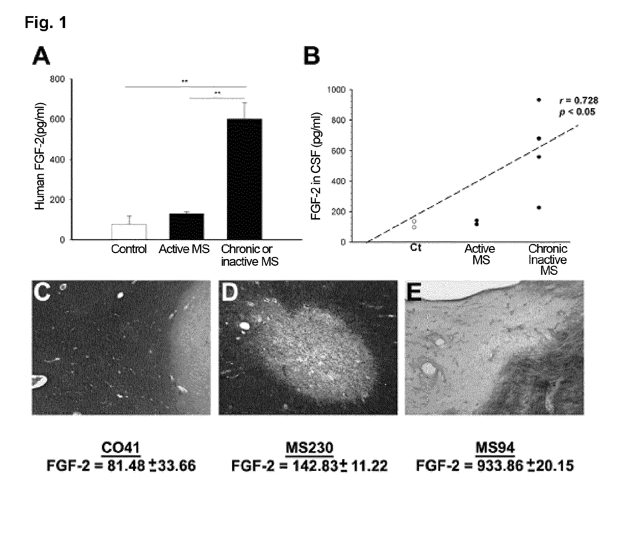

**Figure 1** illustrates how the comparison of the FGF-2 level in the CSF of a multiple sclerosis patient with the level of the control patients shows at a histopathological level the type of demyelination plaques in the white matter. A: Average levels of FGF-2 in CSF of control patients and multiple sclerosis patients. "**" is *p* <0.01 (t Student's t test or Mann-Whithney test for nonparametric samples). B: Correlation between the level of FGF-2 and type of lesions in the white matter of multiple sclerosis patients. Correlation shown according to the Pearson correlation test. C-D: Double immunohistochemical staining for HLD-DR (MHC-II) and Eriochrome cyanin myelin stain of a control patient (C), with an active lesion (D) and an inactive lesion (E). Below, the FGF-2 level shown in the CSF of each of these patients.

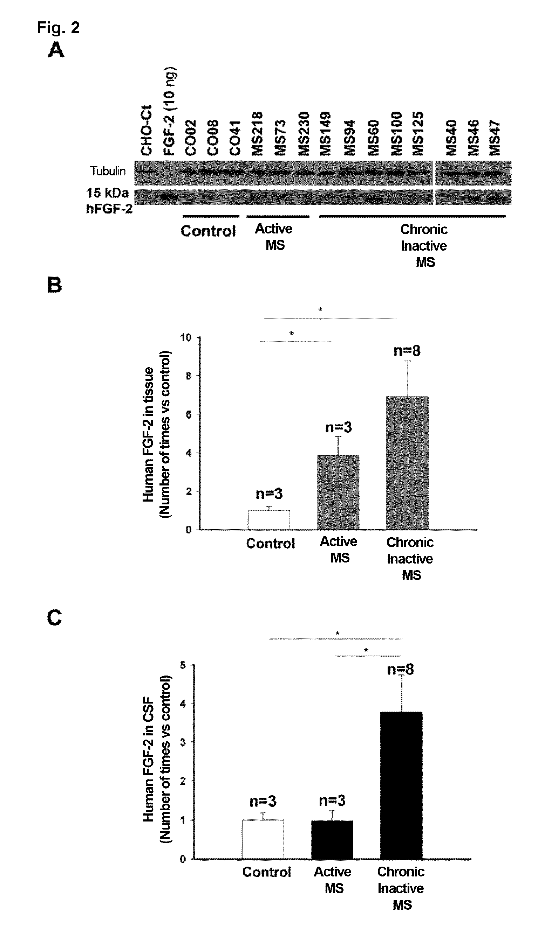

**Figure 2** shows how by only using the measure of the FGF-2 level in the CSF of multiple sclerosis patients it is possible to distinguish between those with only ACTIVE lesions and those with at least one CHRONIC and/or INACTIVE lesion in their CNS. A: Western blot of protein extracts from tissue sections. B: A quantitative analysis of the blot from section A shows that both multiple sclerosis patients who have only active lesions and those with at least one chronic and/or inactive injury have significantly higher levels of FGF-2 than control patients. However, the analysis of the FGF-2 level in these extracts shows no differences between either type of patients. C: The analysis of the FGF-2 level in the CSF of the same patients represented in A-B show significant differences between both groups of multiple sclerosis patients.

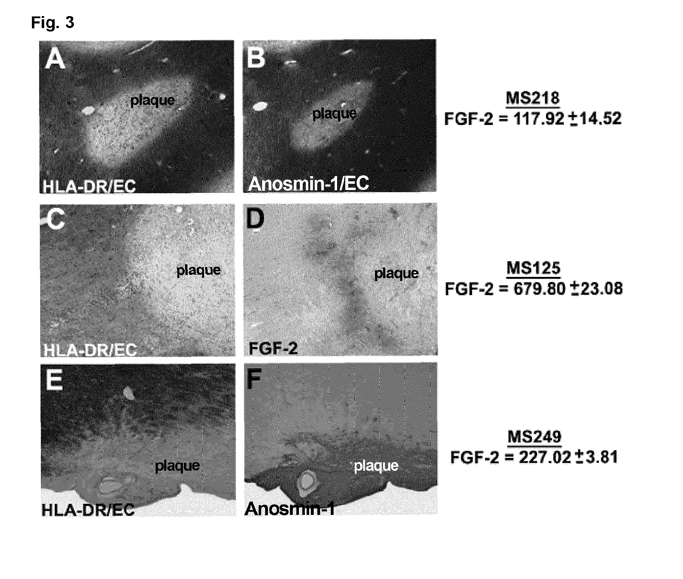

**Figure 3** illustrates how the FGF-2 level in the CSF of a multiple sclerosis patient compared to the level in the control patients reflects the molecular environment associated with the migration of oligodendrocyte precursors. A-B: Double immunohistochemical staining for HLA-DR (A) or Anosmin-1 (B) and Eriochrome cyanin myelin staining (A and B) showing an active lesion that does not have immunolabelling for Anosmin-1. C-D: adjacent sections of a patient with a chronic lesion showing that the lesion area stained with Eriochrome Cyanine and HLA-DR (C) does not present immunolabelling for FGF-2 (D). E-F: Double immunohistochemical staining for HLA-DR (MHC-II) and Eriochrome cyanin myelin staining (E) and simple immunohistochemical staining for Anosmin-1 (F) of a chronic lesion showing how the demyelinating lesion appears filled with Anosmin-1. To the right of each pair of panels the FGF-2 level in the CSF of each of the patients referred to in these pictures is shown.

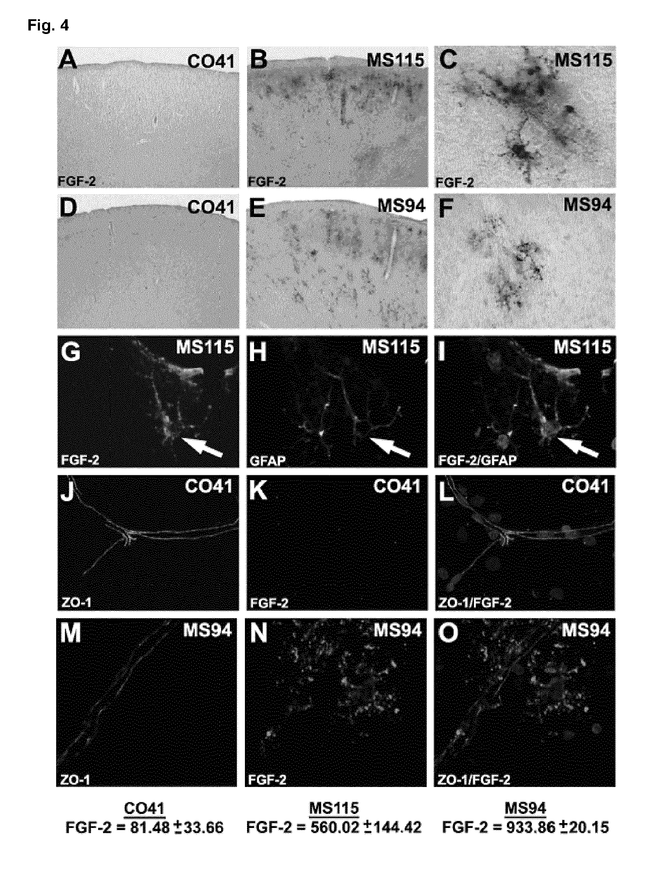

**Figure 4** shows the relationship between the FGF-2 level in the CSF of multiple sclerosis patients compared to that of control patients and the presence of alterations in the grey matter, both as to FGF-2 and Anosmin-1 expression and the integrity of the blood-brain barrier. A-F: Immunohistochemistry for FGF-2 (A-C) and Anosmin-1 (D-F) in the cerebral cortex of a control patient (A, D) and one with multiple sclerosis (B-C, E-F) which shows a strong increase in the staining for FGF-2 and Anosmin-1 in the injured patient, corresponding to perivascular cells. G-I: Example of double-fluorescent immunohistochemistry of these perivascular cells in this case for FGF-2 (G) and GFAP (H) and a mixture of both with a counterstain for nuclei (I) which demonstrates their astrocytic nature. J-O: Double fluorescent immunohistochemistry showing staining for the blood-brain barrier protein ZO-1 (J, M), together with FGF-2 (K, N) and the two mixed together with a counterstain of nuclei (L, O) in the grey matter of a control patient (J-L) and one with chronic and inactive lesions in the white matter (M-O). It is shown how the blood-brain barrier alteration (M) is associated with the appearance of astrocytes positive for FGF-2 (N). The bottom of the figure shows the FGF-2 level in CSF evidenced for each of the patients referred to in the pictures.

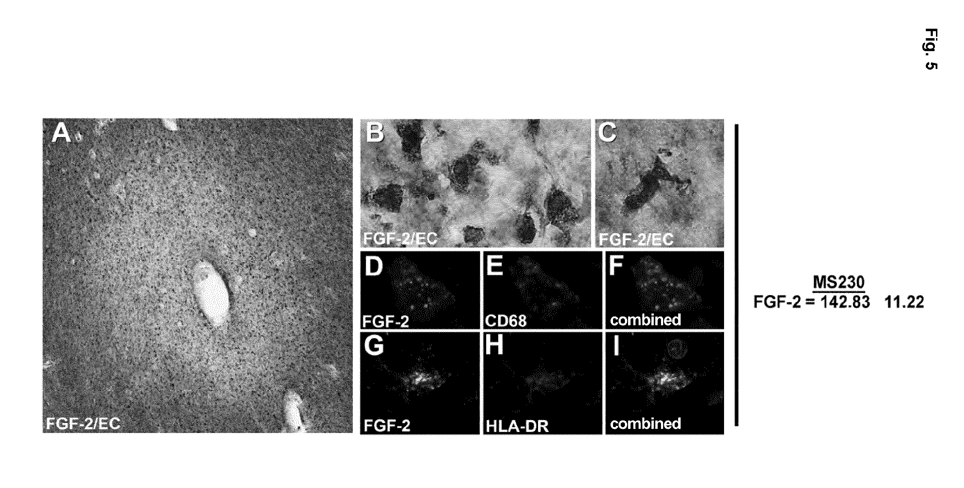

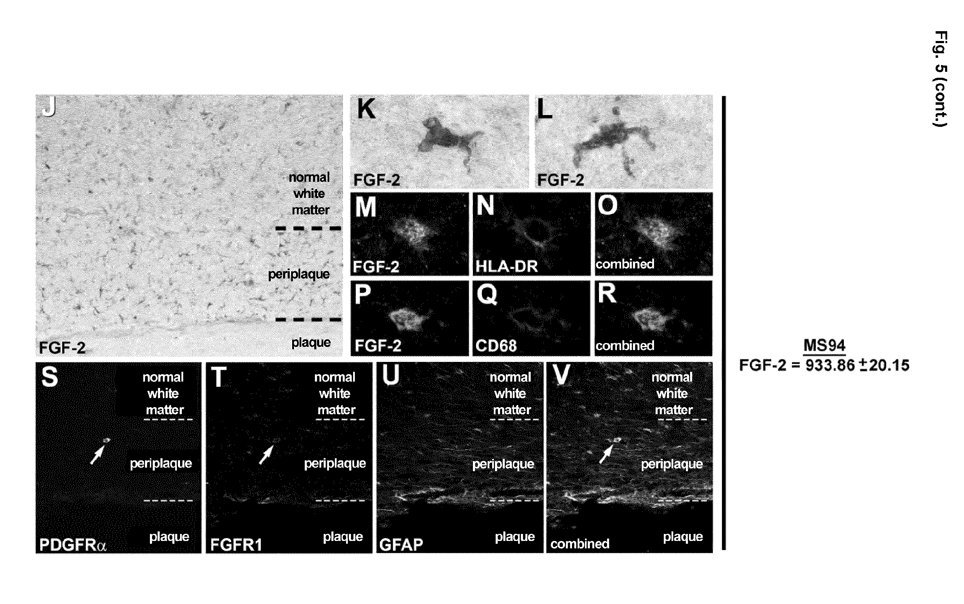

**Figure 5** illustrates how the active lesions only show internal immunolabelled cells for FGF-2 (Figure 5A-C) with microglia/macrophage characteristics and inflammatory cells that appeared doubly labelled for CD68 (Figure 5D-F) and HLA-DR (Figure 5G-I), respectively. By contrast, CHRONIC and INACTIVE lesions showed no marked cells for FGF-2 in its interior but mostly in the periplaque (Figure 5J-L). Similarly, the double immunostaining characterised these cells as immunolabelled inflammatory cells for HLA-DR (Figure 5M-O) with microglia/macrophages characteristics (Fig. 5P-R). Furthermore, CHRONIC or INACTIVE injuries presented precursors of oligodendrocyte immunolabelled with PDGFRα (Figure 5E) in the periplaque, showing immunolabelling for FGFR1 (Figure 5T), but not for GFAP (Fig.5U-V).

### EXAMPLES

The following specific examples provided in this patent document serve to illustrate the nature of the present invention. These examples are included for illustrative purposes only and should not be interpreted as limitations of the invention claimed herein. Therefore, the examples described below illustrate the invention without limiting the scope thereof.

### EXAMPLE 1

In this example, the inventors demonstrate how measuring the FGF-2 level in the CSF of multiple sclerosis patients correlates with the type of lesions histologically found in the cerebral cortex of these patients.

As a source of both tissue and CSF, samples from patients of various ages and with different types of multiple sclerosis were used (Table 1), all from the UK Multiple Sclerosis Tissue Bank, London (United Kingdom).

**Table 1. Summary of cases analysed for this study.**

| Patient | Age/Sex | Diagnosis | TP | CSF | Number of Plaques | Type of lesion¹ | | |
|---|---|---|---|---|---|---|---|---|
| | | | | | | Active | Chronic | Inactive |
| MS40 | 58/F | PP | 6h | √ | 15 | 11 | 3 | |
| MS46 | 40/M | SP | 18h | √ | 13 | 9 | 2 | 1 |
| MS47 | 66/F | SP | 17h | √ | 15 | 5 | | 5 |
| MS60 | 55/M | SP | 16h | √ | 8 | 4 | 1 | 1 |
| MS73 | 80/F | SP | 20h | √ | 8 | 6 | | |
| MS94 | 42/F | PP | 11h | √ | 10 | 5 | 3 | 2 |
| MS100 | 46/F | SP | 7h | √ | 14 | 3 | 5 | 3 |
| MS106 | 39/F | SP | 18h | ∅ | 9 | 3 | 1 | 5 |
| MS125 | 76/F | SP | 13h | √ | 5 | 1 | 3 | |
| MS149 | 82/F | SP | 15h | √ | 8 | 3 | 3 | 1 |
| MS218 | 56/F | SP | 7h | √ | 15 | 13 | | |
| MS230 | 42/F | SP | 31h | √ | 6 | 6 | | |
| MS249 | 59/F | SP | 8h | ∅ | 18 | 14 | | |
| MS342 | 35/F | SP | 9h | ∅ | 13 | 11 | 2 | |
| CO02 | 95/F | Normal | 10h | √ | | | | |
| CO08 | 93/F | Normal | 9h | √ | | | | |
| CO25 | 35/M | Normal | 22h | ∅ | | | | |
| CO30 | 75/M | Normal | 17h | ∅ | | | | |
| CO41 | 54/M | Normal | 20h | √ | | | | |

| | | | | | | | | |
|---|---|---|---|---|---|---|---|---|
| CO: control; F: female; h: hours; MS: multiple sclerosis; PP: primary progressive; SP: secondary progressive; TP: time post-mortem; M: male. 1, √ shows that there is a CSF sample and ∅ shows that there is not. Classification criteria described in Benito et al., J. Neurosci., 2007, vol. 27, pp. 2396-2402. | | | | | | | | |

The classification of lesions was performed by double staining for HLA-DR (MHC-II) and Eriochrome Cyanine histological staining for myelin. 50-µm-thick histological sections were obtained using a sliding microtome coupled to a cold source and were collected and stored until use.

Firstly, after washing, the sections were immersed in a medium containing citrate buffer (pH 6.0) at 90°C for 10 minutes. Subsequently, endogenous peroxidases were inactivated using a 20-minute pre-treatment in phosphate buffer (0.1 M, pH 7.4), with 10% methanol and 3% H₂O₂. They were then pre-incubated for 1 hour in a medium containing phosphate buffered saline (pH 7.4) with 5% (v/v) normal horse serum and 0.2% Triton X-100 (v/v), and were then incubated overnight (4 °C) in this same pre-incubation medium with anti-HLA-DR antibody (1:200). For immunohistochemical detection an anti-mouse secondary antibody was used, followed by the ABC detection method and DAB chromogen in the presence of H₂O₂, to visualise the cells bound to the primary antibody.

Subsequently, the sections were dried overnight at room temperature and 2 hours more at 37ºC, after which they were immersed in acetone for 5 minutes and allowed to dry at room temperature for 30 minutes. The sections were stained for one hour with Eriochrome Cyanine and then washed in running tap water, after which the differentiation process was carried out: i) iron alum 5% (w/v) for 5 minutes, ii) washing with tap water and iii) borax-potassium ferricyanide 1% (w/v) and 1.25% (w/v), respectively, for 10 minutes, controlling the colour under the microscope.

The CSF of multiple sclerosis patients were centrifuged at 14,000 rpm for 5 minutes at 4°C and analysed by using an ELISA kit specific for human FGF-2 with a sensitivity limit of 2 pg/ml.

A summary of the presence of FGF-2 immunodetection in tissue and CSF of multiple sclerosis patients is described in Table 2. The control patients showed no demyelinating lesions (Figure 1A-C). Multiple sclerosis patients whose levels of FGF-2 were similar to those of the control patients showed, in the white matter only, abundant, rounded or oval perivascular lesions, completely full of positive cells to MHC-II, all of them classified as ACTIVE type (Figure 1A-B, D).

**Table 2. Correlation between the presence of FGF-2 in tissue and in CSF.**

| Patient | Type of lesion | | | | FGF-2 immunoreactivity | | |
|---|---|---|---|---|---|---|---|
| | A | C | I | Ast | Pe r | CSF | |
| | | | | | | pg/ml | No. times vs Ct |
| CO02 | - | - | - | - | | 417 ± 5 | 1.51 ± 0.02 |
| C008 | - | - | - | - | | 330 ± 26 | 1.2 ± 0.76 |
| CO41 | - | - | - | - | | 98 ± 14 | 0.30 ± 0.1 |
| MS73 | √ | - | - | - | - | 552 ± 13 | 1.99 ± 0.05 |
| MS218 | √ | - | - | - | - | 118 ± 15 | 0.43 ± 0.04 |
| MS230 | √ | - | - | - | - | 143 ± 11 | 0.52 ± 0.03 |
| MS40 | √ | √ | - | + | + | 375 ± 16 | 1.36 ± 0.08 |
| MS46 | √ | √ | √ | + | + | 67 ± 13 | 0.24 ± 0.07 |
| MS47 | √ | - | √ | + | + | 1314 ± 38 | 4.76 ± 0.1 |
| MS60 | √ | √ | √ | + | + | 3779 ± 277 | 13.68 ± 1.16 |
| MS094 | √ | √ | √ | + | + | 934 ± 20 | 3.38 ± 0.06 |
| MS100 | √ | √ | √ | + | + | 525 ± 43 | 1.90 ± 0.18 |
| MS115 | - | - | - | + | - | 560 ± 14 | 2.09 ± 0.07 |
| MS125 | - | √ | - | + | + | 680 ± 23 | 2.46 ± 0.07 |
| MS149 | √ | √ | √ | + | + | 227 ± 4 | 0.82 ± 0.01 |

| | | | | | | | |
|---|---|---|---|---|---|---|---|
| A: Active lesions. C: Chronic lesions. I: Inactive lesions.√ shows the presence of lesions, while - indicates their absence. Regarding FGF-2 immunoreactivity, Ast: Astrocytes. Per: Periplaque. + shows that the labelling was positive and - negative. | | | | | | | |

Patients whose FGF-2 levels in CSF were much higher than those present in control patients' CSF, always presented a CHRONIC or INACTIVE lesion, notwithstanding them having both or even one of an active type (Figure 1A-B, E). These lesions were characterised by MHC-II positive cells around the lesion, and virtually none inside (chronic lesion), or completely empty of MHC-II positive cells (inactive lesions). These lesions were always large and occurred in low numbers per block of tissue. Furthermore, the presence of one kind of injury or another was independent of the patient's age (*r* = 0.0357, *p* = 0.939), of the post-mortem interval (*r* = -0.321, *p* = 0.483), as well as of the type of multiple sclerosis (see Tables 1 and 2).

### EXAMPLE 2

In this example, the inventors demonstrate how by measuring the FGF-2 level in CSF the presence of two groups of patients can be defined, while they cannot be distinguished when FGF-2 is measured directly from the tissue (WB). Proteins were extracted from histological sections of cerebral cortex using a protein extraction kit from fixed tissue, and the presence of FGF-2 or β-tubulin (as loading control) was immunodetected using the Western blot technique (Figure 2A) hybridising with a primary polyclonal antibody made in goat (1:200) or monoclonal antibody made in mouse (1:30,000) followed by an anti-goat biotinylated secondary antibody made in horse (1:5,000) or anti-mouse polyclonal antibody made in rabbit HRP-conjugated, respectively. In the case of FGF-2, detection was completed by streptavidin-HRP binding. Immunoblot quantification showed that both patients with only ACTIVE lesions such and those who had at least one CHRONIC and/or INACTIVE lesion presented significantly higher FGF-2 levels than control patients (Figure 2B). However, no statistically significant differences were found between both groups of patients. The ELISA analysis (similar to that described for Example 1) of the CSFs in the same patients from which proteins were extracted from the cerebral cortex (Figure 2C) showed that only patients with CHRONIC and/or INACTIVE lesions presented significantly higher FGF-2 levels than those with only ACTIVE lesions and control patients. This showed that the CSF analysis is a suitable method for distinguishing the histopathology present in patients with multiple sclerosis, as both types of patients could be distinguished.

### EXAMPLE 3

In this example, the inventors demonstrate how measuring the FGF-2 and/or Anosmin-1 levels in the CSF of multiple sclerosis patients is a good bioindicator of the molecular environment found in the white matter demyelination lesions.

To verify this, it was analysed using immunohistochemical staining for FGF-2, FGFR1 or Anosmin-1 and Eriochrome cyanin myelin staining in the same section or in adjacent sections from the same tissue block. The protocol used for immunostaining was the same as for Example 1, but with the following variations. For the immunodetection of FGF-2 or FGFR1 in tissue, different polyclonal antibodies made in goat (1:250 v/v) were used, followed by an anti-goat polyclonal antibody made in horse, biotinylated (1:200) and the ABC detection kit. For Anosmin-1 immunostaining, a polyclonal antibody made in rabbit (1:500) was used, which was later bound to a biotinylated polyclonal antibody made in goat (1:200). The process of immunolabeling was similar and was exposed with DAB and H₂O₂.

Detection of FGF-2 in CSF of control patients and multiple sclerosis patients was performed in the same manner as in Example 1.

The control patients showed no immunostaining for any of the antibodies used. Patients with similar FGF-2 levels to control levels, showed no immunostaining for FGF-2, FGFR1 or Anosmin-1 in or around their ACTIVE lesions (Figure 3A-B). However, those patients in whom the level of FGF-2 in CSF was significantly higher than that detected in control patients, showed strong immunostaining around their CHRONIC and/or INACTIVE injuries for both FGF-2 and FGFR1 (Figure 3C-D). In addition, by completely filling the large CHRONIC and/or INACTIVE lesions present in each patient, a strong immunostaining was observed for Anosmin-1 (Figure 3E, F). The active lesions found in the some patients' tissue with a high FGF-2 level in the CSF showed no difference from those observed in multiple sclerosis patients with similar FGF-2 levels in CSF to those of control subjects, indicating that the presence of FGF-2, FGFR1 and Anosmin-1 is specific to the type of chronic or inactive lesion and not to the individual's age, type of multiple sclerosis or post-mortem interval between death and sample collection).

### EXAMPLE 4

In this example, the inventors disclose the identity of FGF-2 and FGFR1- producing cells in ACTIVE lesions on the one hand and in CHRONIC and INACTIVE on the other. Double histological immunostaining was performed using an antibody for FGF-2 rabbit polyclonal (1:200 v/v) together with the inflammatory cell marker HLA-DR (see above examples) and that specific to microglia/macrophages, CD68 (monoclonal antibody made in mouse, 1:200). A triple immunohistochemical staining was also performed for FGFR1 (polyclonal antibody made in rabbit 1:500 v/v), PDGFRα (polyclonal antibody made in rabbit, 1:100 v/v) and GFAP (monoclonal antibody made in mouse; 1: 500 v/v). In the case of FGFR1 immunofluorescence was intensified by using a biotinylated anti-rabbit seconday antibody made in goat, the ABC kit as well as the enhancement kit Tyramide (TSA) and Streptavidin Texas Red (1:500 v/v) . For GFAP visualisation, a mouse polyclonal antibody made in donkey bound to Alexa 633 was used and for PDGFRα a rabbit polyclonal antibody made in donkey bound to Alexa 488. In addition, the nuclei were counterstained with Hoechst.

The results showed that the active lesions only showed immunolabelled cells for FGF-2 in its interior (Figure 5A-C) with microglia/macrophage characteristics and inflammatory cells that appeared doubly labelled for CD68 (Figure 5D-F) and HLA-DR (Figure 5G-I), respectively. By contrast, CHRONIC and INACTIVE lesions showed no labelled cells for FGF-2 in its interior but mostly in the periplaque (Figure 5J-L). Similarly, the double immunostaining characterised these cells as immunolabelled inflammatory cells for HLA-DR (Figure 5M-O) with microglia/macrophage characteristics (Fig. 5P-R). In addition, chronic and inactive lesions presented in the periplaque oligodendrocyte precursors immunolabelled with PDGFRα (Figure 5S), which showed immunolabelling for FGFR1 (Figure 5T), but not for GFAP (Fig. 5U-V). This confirms and extends the data described in Example 3, showing how patients with only ACTIVE lesions present a favourable environment for cell repair whereas those with CHRONIC and/or INACTIVE lesions show such microenvironment around the lesions but not inside, thus preventing repair. By measuring the FGF-2 in patients' CSF, the general favourable/inhibiting environment of a multiple sclerosis patient can be known.

### EXAMPLE 5

In this example, the inventors show how measurement of the FGF-2 level in CSF is correlated with alterations in the grey matter of multiple sclerosis patients, regardless of the presence of demyelinating lesions in the white matter.

In addition to the immunostaining for FGF-2, FGFR1 and Anosmin-1 and the Eriochrome cyanin myelin staining, in a similar way to that described in Examples 1 and 3, a double immunohistochemistry was carried out against FGF-2 or Anosmin-1 and the protein present in the occluding zonules that are part of the blood brain barrier (ZO-1) or glial fibrillary acidic protein (GFAP), a typical marker of astrocytes. GFAP detection was performed with a polyclonal antibody made in rabbit later bound to a secondary polyclonal antibody against rabbit, made in donkey, and was bound to a fluorescent molecule. For the immunodetection of FGF-2, FGFR1 and Anosmin-1 the same protocol described in Examples 1 and 3 was followed, but without being exposed with DAB and H₂O₂, extending the signal using the tyramide technique and, subsequently, adding a streptavidin molecule bound to a fluorescent molecule with a different emission wavelength and away from the fluorescence emitted for the GFAP case. This allowed the detection of cells that emit fluorescence of one or both colours. Due to the special characteristics of the extracellular matrix proteins, to detect Anosmin-1 sections underwent a specific antigen retrieval process to unmask epitopes by using a citrate buffer bath (pH 6.0) at a high temperature and pressure for 3 minutes.

In the case of double immunohistochemistry against FGF-2 or Anosmin-1 and ZO-1, prior to completing the protocol described in the previous paragraph, a pre-incubation was conducted (at 37 º C for one hour) with 1 mg/ml (w/v) of proteinase K and subsequently in acetone (for 30 minutes at -18 º C). For the detection of ZO-1, a polyclonal antibody made in rabbit was used (1:100, v/v), which the following day was bound to a secondary antibody similar to that described for GFAP.

Measurement of FGF-2 in CSF was performed in the same way as for the three examples shown above.

The results revealed that control patients did not show immunolabelling for FGF-2 or Anosmin-1 in the grey matter (Figure 4A, D) or alterations in blood-brain barrier marked with the anti-ZO-1 antibody (Figure 4J-L). Multiple sclerosis patients whose levels of FGF-2 in CSF were similar to those of control subjects showed no changes in the expression pattern of either marker. By contrast, multiple sclerosis patients with much higher FGF-2 levels in CSF than those of control subjects showed positive cells to FGF-2, FGFR1 and Anosmin-1 around the vessels of the grey matter (Figure 4B-C, E-F), which were identified as perivascular astrocytes by double immunohistochemistry against GFAP (Fig. 4G-I). Moreover, these new cells were also present in those blocks of tissue where there were no demyelinating plaques, the so-called normal-appearing-white-matter. The analysis of the expression pattern of the blood-brain barrier protein ZO-1 showed that these patients' grey matter, including the block without demyelinating plaques, showed a discontinuous pattern, in this case appearing intermittently and unevenly, reflecting the disruption of the blood-brain barrier of the grey matter (Figure 4M-O). In addition, the major changes seen in the ZO-1 expression spatially coincided with the areas of positive astrocytes for FGF-2, indicating an up-regulation of this molecule in order to achieve the maintenance of the barrier. Therefore, measuring the FGF-2 level in CSF is a good bioindicator of the state of impairment of the blood-brain barrier of the grey matter, even in regions where demyelination has not occurred, which may serve as a prognostic test to avoid future neurodegeneration.

## Claims

1. Use of the FGF-2 protein, the Anosmin-1 protein or the combination of both, to predict the histopathology of the lesions of a subject with a possible demyelinating disease, preferably of the central nervous system (CNS), by detecting the amount of such protein in a sample of isolated biological fluid.

2. Use of the FGF-2 protein according to the preceding claim to predict the histopathology of the lesions of a subject with a possible demyelinating disease, preferably of the CNS, by detecting the amount of this protein in a sample of isolated biological fluid.

3. Use of the FGF-2 protein according to any of the preceding claims, wherein the isolated biological fluid sample is a body fluid, and preferably is selected from the list comprising: cerebrospinal fluid (CSF), blood, blood serum, blood plasma and tears.

4. Use of the FGF-2 protein according to the preceding claim, wherein the isolated biological fluid sample is CSF.

5. Use of the FGF-2 protein according to any of the preceding claims, wherein the demyelinating disease affects the CNS and is selected from the list that includes: multiple sclerosis, Devic's neuromyelitis optica, acute disseminated encephalitis, acute transverse myelitis, acute or subacute hemorrhagic leukoencephalitis, acute disseminated demyelination, diffuse sclerosis, central demyelination of the corpus callosum, central pontine myelinolysis, sub-acute necrotising myelitis, concentric sclerosis, adrenoleukodystrophy, Alexander disease, Canavan disease, Krabbe disease and Zellweger syndrome.

6. Use of the FGF-2 protein according to the preceding claim, wherein the CNS demyelinating disease is multiple sclerosis.

7. Use of the FGF-2 protein according to the preceding claim, wherein the CNS demyelinating disease is primary progressive multiple sclerosis, relapsing-remitting multiple sclerosis, progressive relapsing-remitting multiple sclerosis, secondary progressive multiple sclerosis, clinically isolated syndrome (CIS) or multiple sclerosis in any of its clinical presentations, preferably being primary progressive multiple sclerosis or secondary progressive multiple sclerosis

8. Use of the FGF-2 protein according to any of the preceding claims 1 to 4, wherein the demyelinating disease affects the peripheral nervous system (PNS), preferably being the Guillain-Barré syndrome.

9. Use according to any of the preceding claims, **characterised in that** the Anosmin-1 protein is also used as a biomarker for the detection of a demyelinating disease, preferably of the CNS.

10. Use according to the preceding claim, **characterised in that** the Anosmin-1 protein level is also detected in an isolated biological sample, preferably an isolated biological fluid.

11. Method for obtaining useful data to predict the histopathology of the lesions of a subject or a patient with a CNS demyelinating disease, which comprises the following steps:
a) obtaining an isolated biological sample from a subject, preferably an isolated biological fluid;
b) detecting the amount of FGF-2 protein in the biological sample of step (a); and
c) comparing the amount detected in step (b) with a reference quantity.

12. Method according to the preceding claim, further comprising the following step:
d) predicting the presence of at least one chronic or inactive lesion in the white matter and/or the existence of blood-brain barrier damage of the grey matter.

13. Method according to the preceding claim, wherein in step (d) an amount of FGF-2 protein detected in step (b) being greater than the reference amount with which it is compared in step (c) is indicative of the presence of at least one chronic or inactive lesion in the white matter and/or the existence of blood-brain barrier damage of the grey matter.

14. Method according to any of the three preceding claims, wherein the isolated biological sample is a biological body fluid, and is preferably selected from the list that includes: CSF, blood, blood serum, blood plasma and tears.

15. Method according to any of the four preceding claims, wherein the demyelinating disease affects the CNS and is selected from the list that includes: multiple sclerosis, Devic's neuromyelitis optica, acute disseminated encephalitis, acute transverse myelitis, acute or sub-acute hemorrhagic leukoencephalitis, acute disseminated demyelination, diffuse sclerosis, central demyelination of the corpus callosum, central pontine myelinolysis, sub-acute necrotising myelitis, concentric sclerosis, adrenoleukodystrophy, Alexander disease, Canavan disease, Krabbe disease and Zellweger syndrome.

16. Method according to the preceding claim, where the CNS demyelinating disease is multiple sclerosis.

17. Method according to the preceding claim, wherein the CNS demyelinating disease is primary progressive multiple sclerosis, relapsing remitting multiple sclerosis, progressive relapsing-remitting multiple sclerosis, secondary progressive multiple sclerosis, clinically isolated syndrome (CIS) or multiple sclerosis in any of its clinical presentations, preferably being primary progressive multiple sclerosis or secondary progressive multiple sclerosis

18. Method according to any of the preceding claims 11 to 14, wherein the demyelinating disease affects the PNS, preferably being the Guillain-Barré syndrome.

19. Method according to any of the preceding claims 11 to 18, wherein the detection of the amount of FGF-2 protein is carried out by means of an immunoassay.

20. Method according to the preceding claim, wherein the immunoassay is an enzyme-linked immunosorbent assay (ELISA).

21. Method according to any of the preceding claims 11 to 20, wherein the Anosmin-1 protein level is also detected and is compared with a reference quantity in an isolated biological sample, preferably an isolated biological fluid from the subject, where the sample may be the same or different from the sample used for the detection of the FGF-2 protein.

22. Kit for carrying out any of the methods as defined in any of the preceding claims.

23. Use of the Anosmin-1 protein as a biomarker for the detection of a demyelinating disease, preferably of the CNS.

24. Use of the Anosmin-1 protein according to the preceding claim to detect a demyelinating disease in an isolated biological sample, preferably in an isolated biological fluid.

25. Use of the Anosmin-1 protein according to any of the preceding claims, to predict the histopathology of the lesions of a subject with a possible demyelinating disease, preferably of the CNS, by detecting the amount of this protein in a sample of isolated biological fluid.

26. Use of the Anosmin-1 protein according to any of the three preceding claims, wherein the isolated biological fluid sample is a body fluid and is preferably selected from the list that includes: CSF, blood, blood serum, blood plasma and tears.

27. Use of the Anosmin-1 protein according to the preceding claim, wherein the isolated biological fluid sample is CSF.

28. Use of the Anosmin-1 protein according to any of the preceding claims 23 to 27, wherein the demyelinating disease affects the CNS and is selected from the list that includes: multiple sclerosis, Devic's neuromyelitis optica, acute disseminated encephalitis, transverse myelitis, acute or sub-acute hemorrhagic leukoencephalitis, acute disseminated demyelination, diffuse sclerosis, central demyelination of the corpus callosum, central pontine myelinolysis, sub-acute necrotising myelitis, concentric sclerosis, adrenoleukodystrophy, Alexander disease, Canavan disease, Krabbe disease and Zellweger syndrome.

29. Use of the Anosmin-1 protein according to the preceding claim, wherein the CNS demyelinating disease is multiple sclerosis.

30. Use of the Anosmin-1 protein according to the preceding claim, wherein the CNS demyelinating disease is primary progressive multiple slcerosis, relapsing-remitting multiple sclerosis, progressive relapsing-remitting multiple sclerosis, secondary progressive multiple sclerosis, clinically isolated syndrome (CIS) or multiple sclerosis in any of its clinical presentations, preferably being primary progressive multiple sclerosis or secondary progressive multiple sclerosis.

31. Use of the Anosmin-1 protein according to any of the preceding claims 23 to 27, wherein the demyelinating disease affects the PNS, preferably being the Guillain-Barré syndrome.

32. A method for obtaining useful data for the detection of a demyelinating disease or to predict the histopathology of the lesions of a subject or a patient with a CNS demyelinating disease, comprising the following steps:
a) obtaining an isolated biological sample from a subject, preferably an isolated biological fluid;
b) detecting the amount of Anosmin-1 protein in the biological sample of step (a), and
c) comparing the amount detected in step (b) with a reference quantity.

33. Method according to the preceding claim, further comprising the following step:
d) predicting the presence of at least one chronic or inactive lesion in the white matter and/or the existence of blood-brain barrier damage in the grey matter.

34. A method according to the preceding claim, wherein in step (d) an amount of Anosmin-1 protein detected in step (b) being greater than the reference amount with which it is compared in step (c) is indicative of the presence at least one chronic or inactive lesion in the white matter and/or the existence of blood-brain barrier damage in the grey matter.

35. A method according to any of the three preceding claims, wherein the isolated biological sample is a biological body fluid, and is preferably selected from the list comprising: CSF, blood, blood serum, blood plasma and tears.

36. A method according to any of the four preceding claims, wherein the demyelinating disease affects the CNS and is selected from the list comprising: multiple sclerosis, Devic's neuromyelitis optica, acute disseminated encephalitis, acute transverse myelitis, acute or sub-acute hemorrhagic leukoencephalitis, acute disseminated demyelination, diffuse sclerosis, central demyelination of the corpus callosum, central pontine myelinolysis, sub-acute necrotising myelitis, concentric sclerosis, adrenoleukodystrophy, Alexander disease, Canavan disease, Krabbe disease and Zellweger syndrome.

37. A method according to the preceding claim, wherein the CNS demyelinating disease is multiple sclerosis.

38. A method according to the preceding claim, wherein the CNS demyelinating disease is primary progressive multiple sclerosis, relapsing-remitting multiple sclerosis, progressive relapsing-remitting multiple sclerosis, secondary progressive multiple sclerosis, clinically isolated syndrome (CIS) or multiple sclerosis in any of its clinical presentations, preferably being primary progressive multiple sclerosis or secondary progressive multiple sclerosis.

39. A method according to any of the preceding claims 32 to 35, wherein the demyelinating disease affects the PNS, preferably being the Guillain-Barré syndrome.

40. A method according to any of the preceding claims 32 to 39, wherein detection of the amount of Anosmin-1 protein is carried out by means of an immunoassay.

41. A method according to the precedinf claim, wherein the immunoassay is an enzyme-linked immunosorbent assay (ELISA).

42. Kit for carrying out any of the methods as defined in any of the preceding claims 32 to 41.
